(19) Europäisches Patentamt
European Patent Office
Office européen des brevets

(11) Publication number: 0 361 772 A2

# (12) EUROPEAN PATENT APPLICATION

(21) Application number: 89309552.1

(51) Int. Cl.[5]: C07C 405/00 , A61K 31/557

(22) Date of filing: 20.09.89

(30) Priority: 21.09.88 GB 8822141

(43) Date of publication of application: 04.04.90 Bulletin 90/14

(84) Designated Contracting States: AT BE CH DE ES FR GB GR IT LI LU NL SE

(71) Applicant: GLAXO GROUP LIMITED
Clarges House 6-12 Clarges Street
London W1Y 8DH(GB)

(72) Inventor: Collington, Eric William
28 Watton Road
Knebworth Hertfordshire(GB)
Inventor: Swanson, Stephen
23 Kiln House Close
Ware Hertfordshire SG12 7ES(GB)
Inventor: Finch, Harry
31 Newlands
Letchworth Hertfordshire(GB)
Inventor: Capps, Nigel Kenneth
12 Whiteley Close Dane End
Ware Hertfordshire SG12 0NB(GB)
Inventor: Humphrey, Patrick Paul Anthony
Arcadia House Church Lane
Wrestlingworth Bedfordshire SG19 2EU(GB)

(74) Representative: Skailes, Humphrey John et al
Frank B. Dehn & Co. Imperial House 15-19 Kingsway
London WC2B 6UZ(GB)

## (54) Cyclopentyl ethers and their preparation and pharmaceutical formulation.

(57) Compounds of the general formula (I)

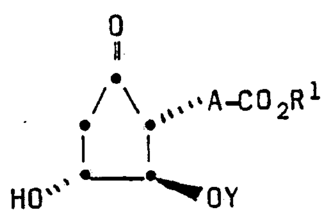

(I)

and the physiologically acceptable solvates and cyclodextrin complexes thereof, in which
$R^1$ represents phenyl substituted by a group $-NHCOR^5$ (where R5 is substituted phenyl);
A is $-(CH_2)_nX(CH_2)_m-$ or $-(CH_2)_pS(O)_q(CH_2)_r-$ (where n is 1 or 2, m is 2-5 and X is cis or trans -CH=CH- or $-CH_2CH_2-$ or m is 1-4 and X is -CH=C=CH-; p is 1-4, q is zero, 1 or 2 and r is 1-4);
Y is substituted or unsubstituted 3-phenoxy-2-hydroxypropyl.

These compounds inhibit gastric acid secretion and provide gastrointestinal cytoprotection and may be formulated for use in the treatment of ulcers. They may also be of use in the therapy or prophylaxis of atherosclerosis and other disorders associated with abnormal levels of blood lipids and serum chlolesterol.

EP 0 361 772 A2

## CYCLOPENTYL ETHERS AND THEIR PREPARATION AND PHARMACEUTICAL FORMULATION

This invention relates to a new group of cyclopentyl ethers and their use in chemotherapy. More specifically the invention is concerned with cyclopentyl ethers having $PGE_2$-type activity, the preparation thereof, pharmaceutical formulations containing such compounds and their use in the treatment of ulcers and conditions where controlling non-esterified fatty acids and triglycerides and therefore lipoproteins and cholesterol would be beneficial.

Thus, according to one aspect of the invention, we provide compounds of the general formula (I)

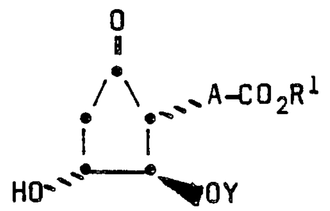

(I)

and the physiologically acceptable solvates and cyclodextrin complexes thereof, wherein :
$R^1$ represents phenyl substituted by a group $-NHCOR^5$ [where $R^5$ is phenyl substituted by a group selected from $-C{\equiv}CR^6$ (where $R^6$ is hydrogen, $C_{1-6}$ alkyl or phenyl), phenyl$C_{1-6}$alkyl or alkyl (e.g. $C_{1-10}$ alkyl) optionally substituted by hydroxy, $-NHSO_2R^7$, $-NHCOR^7$ or $-CONR^8R^9$ (where $R^7$ is $C_{1-4}$alkyl or phenyl and $R^8$ and $R^9$ may be the same or different and are each a hydrogen atom or a $C_{1-4}$ alkyl group)];
A represents a group $-(CH_2)_nX(CH_2)_m-$ or a group $-(CH_2)_pS(O)_q(CH_2)_r-$ (where n is 1 or 2, m is 2-5 and X is cis or trans $-CH=CH-$ or $-CH_2CH_2-$ or m is 1-4 and X is $-CH=C=CH$; p is 1-4, q is zero, 1 or 2 and r is 1-4); and
Y is

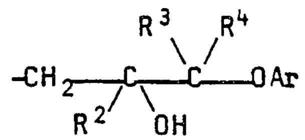

(where $R^2$, $R^3$ and $R^4$ are each a hydrogen atom or a methyl group and at least one of $R^2$, $R^3$ and $R^4$ is a hydrogen atom and Ar represents a phenyl group optionally substituted by one or two $C_{1-4}$alkyl, $C_{1-4}$alkoxy, $C_{1-4}$alkylthio, $C_{1-4}$alkylsulphinyl, $C_{1-4}$alkylsulphonyl, halogen or trifluoromethyl groups).

It will be understood that the present invention encompasses the individual stereoisomers (including diastereoisomers) of the compounds of formula (I) as well as mixtures (including racemic mixtures) thereof.

The alkyl groups referred to above in the definition of compounds of the invention may be straight or branched chain groups. When the substituent on the $R^5$ phenyl ring contains a phenyl group this phenyl group may be substituted by one or more halogen atoms or $C_{1-4}$ alkoxy or $C_{1-4}$ alkyl groups. The term "halogen" means herein fluorine, chlorine, bromine or iodine.

In general, compounds of formula (I) in which the carbon atom carrying the group $-A-CO_2R^1$ and/or the carbon atom in the group Y carrying the hydroxyl group (particularly the former) are in the R-configuration and mixtures containing such isomers are preferred.

$R^1$ preferably represents phenyl substituted in the para position by the group $-NHCOR^5$.

In compounds of formula (I) where A represents a group $-(CH_2)_nX(CH_2)_m-$, when X is $-CH=CH-$ or $-CH_2CH_2-$ then m is preferably 3 when n is 1 and m is preferably 2 or 4 when n is 2. When X is $-CH=C=CH-$ then m is preferably 2 when n is 1 and m is preferably 1 or 3 when n is 2. When X is $-CH=CH-$ it is preferably cis-$CH=CH-$.

In compounds of formula (I) where A represents a group $-(CH_2)_pS(O)_q(CH_2)_r-$, q is preferably zero, p is preferably 3 or 4 when r is 1, p is preferably 2 or 3 when r is 2, p is preferably 2 when r is 3 and p is preferably 1 when r is 4.

Compounds of formula (I) in which A represents $-(CH_2)_nX(CH_2)_m-$ are generally preferred.

In the group Y, $R^3$ and $R^4$ are preferably hydrogen atoms. When the Ar phenyl group is substituted, the substituent may be for example methyl, ethyl, propyl, butyl, ethoxy, propoxy, butoxy, methylthio, methylsulphinyl, methylsulphonyl, fluoro, chloro, bromo or trifluoromethyl. Preferably, only a single substituent is present on the Ar phenyl ring and this is preferably attached at the para position. In general, Ar is preferably phenyl or phenyl substituted by halogen, particularly fluoro or chloro. Compounds of formula (I) in which Ar is phenyl are particularly preferred.

$R^5$ preferably represents phenyl substituted by $-C\equiv CR^6$ (where $R^6$ is $C_{1-4}$ alkyl, e.g. butyl, or phenyl), phenyl$C_{1-3}$alkyl or $C_{1-10}$ alkyl (e.g. $C_{4-8}$alkyl) substituted by either hydroxy, $-NHSO_2R^7$, $-NHCOR^7$ or $-CONR^8R^9$. The substituent on the $R^5$ phenyl ring is preferably attached at the para position relative to the rest of the molecule. $R^7$ is preferably phenyl and $R^8$ and $R^9$ are preferably each hydrogen atoms.

The preferences indicated above apply both separately or in combination with one or more of the other stated preferences.

A preferred group of compounds of the invention are those compounds of formula (I) and their physiologically acceptable solvates and cyclodextrin complexes wherein :

A represents a group $-(CH_2)_nX(CH_2)_m-$ (where n, m and X are as defined above) and is especially $-CH_2CH=CH(CH_2)_3-$ or $-CH_2CH_2CH=CHCH_2CH_2-$;

$R^1$ represents

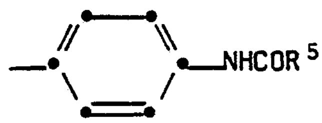

(where $R^5$ is as defined above); and

Y represents

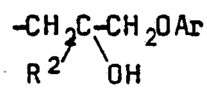

(where $R^2$ is a hydrogen atom or a methyl group and Ar is phenyl or phenyl substituted by fluoro or chloro). Compounds of this type in which the carbon atom carrying the $-A-CO_2R^1$ group is in the R-configuration are particularly preferred.

Particularly preferred compounds of the invention are those in which A represents $-CH_2CH_2CH=CHCH_2CH_2-$ wherein the double bond is in the trans, or more preferably, the cis configuration.

A particularly useful group of compounds according to the invention are the following :

[1R-[1α(Z),2β(2R*),3α]]-4-[4-[6-(benzoylamino)hexyl]benzoylamino]phenyl 7-[3-hydroxy-2-(2-hydroxy-3-phenoxypropoxy)-5-oxocyclopentyl]-4-heptenoate;

[1R-[1α(Z),2β(2R*),3α]]-4-(phenylethynyl) benzoylamino]phenyl7-[3-hydroxy-2-(2-hydroxy-3-phenoxypropoxy)-5-oxocyclopentyl]-4-heptenoate;

[1R-[1α(Z),2β(R*),3α]]-4-[4-(2-phenylethyl)benzoylamino]phenyl 7-[3-hydroxy-2-(2-hydroxy-3-phenoxypropoxy)-5-oxocyclopentyl]-4-heptenoate; and the physiologically acceptable solvates and cyclodextrin complexes thereof.

A particularly preferred compound according to the invention is :

[1R-[1α(Z),2β(2R*),3α]]-4-[4-[6-[(phenylsulphonyl)amino]hexyl]benzoylamino]phenyl 7-[3-hydroxy-2-(2-hydroxy-3-phenoxypropoxy)-5-oxocyclopentyl]-4-heptenoate; and its physiologically acceptable solvates and cyclodextrin complexes.

Compounds of formula (I) inhibit gastric secretion, as determined for example by their ability to inhibit histamine-induced secretory responses in the rat perfused stomach, following the method of Ghosh and Schild in Br.J.Pharmacol., 1958, 13, 54 as modified by Parsons M.E., Ph.D Thesis, University of London, 1969.

The compounds also provide gastrointestinal cytoprotection, as determined for example by their ability to inhibit ethanol-induced lesions in the conscious rat, following the method of Robert et al in Gastroenterology, 1979, 77, 433, modified by the use of 5mg/kg/s.c. indomethacin prior to the administration of the test compound.

Compounds of the invention are also able to lower lipid levels as may be demonstrated in standard animal models, for example by determining their ability to lower non-esterified fatty acid levels and triglyceride levels in the starved rat. (P. P. Lovisolo et. al., Pharmacological Research Communications,

1981, 13, 163-174; E. Schillinger and O. Loge, Biochemical Pharmacology, 1974, 23, 2283-2289)

The compounds are thus of interest for use in therapy, more particularly in the prevention and/or treatment of ulcers. They may also be used in the treatment of other conditions which arise from the hypersecretion of gastric acid. They may also be used for the prevention and/or treatment of conditions associated with lipid imbalance or abnormalities in lipid metabolism including hyperlipidemia, hypercholesterolemia, atherosclerosis, peripheral vascular disease and other cardiovascular conditions, and diabetes mellitus.

According to a further aspect of the present invention we therefore provide a compound of formula (I) or a physiologically acceptable solvate or cyclodextrin complex thereof for use in the prevention and/or treatment of ulcers and other conditions arising from hypersecretion of gastric acid. We also provide a compound of formula (I) or a physiologically acceptable solvate or cyclodextrin complex thereof for use in the prevention and/or treatment of atherosclerosis or other diseases associated with lipid imbalance or abnormalities in lipid mechanism such as hyperlipidemia, hypercholesterolemia, peripheral vascular disease and other cardiovascular conditions, and diabetes mellitus.

According to another aspect of the invention we provide the use of a compound of formula (I) or a physiologically acceptable solvate or cyclodextrin complex thereof in the manufacture of a medicament for the prevention or treatment of ulcers and other conditions arising from hypersecretion of gastric acid. We also provide the use of a compound of formula (I) or a physiologically acceptable solvate or cyclodextrin complex thereof in the manufacture of a medicament for the prevention or treatment of atherosclerosis and other diseases associated with lipid imbalance or abnormalities in lipid mechanism.

According to a further aspect of the invention we provide a method of treating the human or non-human animal body to combat ulcers and other conditions arising from hypersecretion of gastric acid or conditions such as atheroscelerosis or other diseases associated with lipid imbalance or abnormalities in lipid mechanism such as hyperlipidemia, hypercholesterolemia, peripheral vascular disease and other cardiovascular conditions, and diabetes mellitus, which method comprises administering to the said body an effective amount of a compound of formula (I) or a physiologically acceptable solvate or cyclodextrin complex thereof.

It will be appreciated that the compounds according to the invention may advantageously be used in conjunction with one or more other therapeutic agents, such as non-steroidal anti-inflammatory agents, or different anti-ulcer agents. It is to be understood that the present invention covers the use of a compound of formula (I) or a physiologically acceptable solvate or cyclodextrin complex thereof in combination with one or more other therapeutic agents.

While it is possible that, for use in therapy, the compounds of formula (I) may be administered as the raw compound it is preferably to the present the active ingredient as a pharmaceutical formulation.

In a further aspect of the present invention therefore we provide a pharmaceutical composition comprising as an active ingredient a compound of formula (I) or a physiologically acceptable solvate or cyclodextrin complex thereof together with one or more pharmaceutical carriers or excipients.

Compounds may be formulated in conventional manner with one or more pharmaceutical carriers, for example for oral, buccal, parenteral or rectal administration.

The compounds may be formulated for oral administration as, for example, tablets, capsules, powders, solutions or syrups prepared by conventional means with acceptable excipients.

The compounds may be formulated for parenteral administration by bolus injections or continuous infusion. Formulations for injections may be presented in unit dosage form in ampoules, or in multi-dose containers, with an added preservative.

For buccal administration, the compounds may be formulated as tablets or lozenges in conventional manner; and for rectal administration compositions such as suppositories or retention enemas, for example containing conventional suppository bases such as cocoa butter or other glyceride, can be used.

The compounds are preferably administered orally, for example in amounts of 0.1 to 500 µg/kg body weight, preferably 0.1 to 100µg/kg body weight, 1 to 4 times daily. For parenteral administration, the compounds may be administered in amounts of 0.01 to 100µg/kg body weight, 1 to 4 times daily. The precise dose will however of course depend on the age and condition of the patient, the ailment to be treated and will ultimately be at the discretion of the attendant physician.

The desired dose may conveniently be presented as a single dose or as divided doses administered at appropriate intervals, for example as two, three, four or more sub-doses per day.

The compound is conveniently administered in unit dosage form; for example containing 5µg to 35mg, conveniently 5µg to 10mg of active ingredient per unit dose when administered orally and containing, for example, 0.5µg to 10mg of active ingredient per unit dose when administered parenterally.

Suitable methods for preparing the compounds of the invention are described below, the various groups

and symbols being as defined above except where otherwise indicated.

(a) Compounds of formula (I) may be prepared by deprotection of a corresponding compound of formula (II)

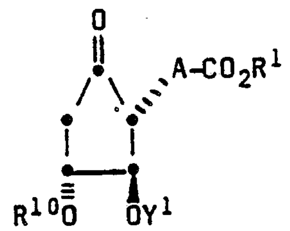

(II)

in which $Y^1$ is defined as a group

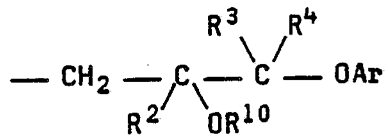

and $R^{10}$ is a hydroxyl protecting group.

The two $R^{10}$ groups in the compounds of formula (II) are conveniently the same, but they may be different if desired.

Suitable hydroxyl protecting groups for $R^{10}$ are described in 'Protective Groups in Organic Chemistry', Ed. J. F. W. McOmie (Plenum Press, 1973) and 'Protective Groups in Organic Synthesis' by Theodora W. Greene (John Wiley and Sons, 1981). Examples of suitable hydroxyl protecting groups include tetrahydropyran-2-yl, tetrahydrofuran-2-yl, ethoxyethyl, tri(hydrocarbyl)silyl and arylmethyl.

Where $R^{10}$ is tri(hydrocarbyl)silysl the hydrocarbyl substituents may be the same or different e.g. $C_{1-6}$ alkyl, $C_{2-6}$ alkenyl, $C_{3-7}$ cycloalkyl, $C_{7-20}$ aralkyl and $C_{6-20}$ aryl groups. Such groups include methyl, ethyl, n-propyl, isopropyl, n-butyl, isobutyl, t-butyl, allyl, phenyl and benzyl. Preferred hydrocarbyl groups are $C_{1-4}$ alkyl, e.g. methyl and t-butyl. Trimethylsilyl and t-butyldimethylsilyl groups are particularly preferred.

When $R^{10}$ is an arylmethyl group it may contain up to 20 carbon atoms, e.g. benzyl, diphenylmethyl or triphenylmethyl.

The method used to deprotect the protected hydroxyl group will depend on the nature of $R^{10}$ but in general acid hydrolysis or reduction may be used.

Thus, for example when $R^{10}$ is a tetrahydropyran-2-yl, tetrahydrofuran-2-yl or ethoxyethyl group deprotection may be carried out with an acid. Suitable acids include inorganic acids such as hydrochloric acid and organic acids such as acetic acid or trifluoroacetic acid. Suitable solvents include ethers (e.g. diethyl ether, dioxan and tetrahydrofuran), halogenated hydrocarbons (e.g. dichloromethane), hydrocarbons (e.g. toluene), dipolar aprotic solvents (e.g. acetone, acetonitrile, dimethylsulphoxide and dimethylformamide) and alcohols (e.g. methanol, ethanol and ethylene glycol). Where desired the solvents may be used in combination with water. The reaction may be carried out at any suitable temperature, such as from 0° to 50° C, e.g. 40° to 50° C.

A tri(hydrocarbyl)silyl group may for example be removed by acid hydrolysis, e.g. with dilute mineral acid or trifluoroacetic acid or by treatment with fluoride ions (e.g. from a quaternary ammonium fluoride such as tetra-n-butyl ammonium fluoride), or by treatment with aqueous hydrogen fluoride. Arylmethyl groups may be removed by reduction, e.g. by hydrogenolysis, e.g. with a noble metal catalyst such as platinum or palladium, or by treatment with a Lewis acid (e.g. boron trifluoride-etherate) in the presence of a thiol (e.g. ethanethiol) in a suitable solvent such as dichloromethane at e.g. room temperature.

It will be appreciated that when $R^1$ contains a hydroxyl group this group may also be protected by one of the aforementioned groupings, and the protecting group removed when removal of the $R^{10}$ groups is effected and under the same conditions.

Compounds of formula (II) may be prepared by oxidation of a compound of formula (III)

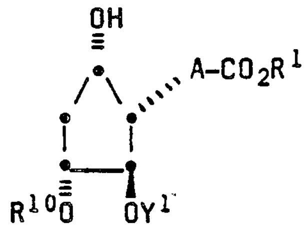

(III)

(where $Y^1$ and $R^{10}$ are as defined above) with for example pyridinium chlorochromate in the presence of a buffer (e.g. sodium acetate) in a suitable solvent (e.g. dichloromethane) at an appropriate temperature (e.g. -10 °C to room temperature). Alternatively, the oxidation may be carried out with dimethylsulphoxide, activated by N,N′-dicyclohexylcarbodiimide, in the presence of pyridinium trifluoroacetate in a solvent such as dichloromethane at e.g. -10 °C to room temperature. Other conventional oxidative methods can also be used, for example Jones reagent.

Intermediate compounds of formula (III) may be prepared from the corresponding known acids (i.e. compounds of formula (III) in which $R^1$ represents a hydrogen atom) by esterification according to the methods generally described in GB-A-2174702 and EP-A-265247 or the methods described hereinafter. The acid precursors to the compounds of formula (III) are described in GB-A-2174702 and EP-A-265247.

It will be appreciated that the deprotection method (a) is usually applied in connection with the formation by oxidation of the cyclopentyl ring oxo group. Thus, the compounds of formula (I) may generally be prepared by oxidising a corresponding compound of formula (III) and the protecting groups removed thereafter.

The formation of the ring oxo group may however be effected prior to the introduction of the desired $R^1$ group by esterification (e.g. by method (b) below) and the protecting groups removed thereafter. (b) Compounds of formula (I) may also be prepared by esterifying the corresponding carboxylic acids, (i.e. the compounds in which $R^1$ is a hydrogen atom), by conventional methods.

Thus for example a compound of formula (I) may be prepared by conversion of the corresponding carboxylic acid into an activated derivative (e.g. a corresponding mixed anhydride) formed for example by reaction with an alkyl chloroformate (e.g. isobutyl chloroformate) or an acid chloride (e.g. pivaloyl chloride) in the presence of a suitable base (e.g. triethylamine or pyridine). The activated derivative can then be reacted with an appropriate compound $R^1OH$, which are either known compounds or may be prepared by methods analogous to those used for the preparation of known compounds. Suitable solvents include dipolar aprotic solvents (e.g. acetone, acetonitrile and dimethylformamide) and halogenated hydrocarbons (e.g. dichloromethane). The reaction may be carried out at any suitable temperature e.g. from 0 °C to room temperature.

The same group of compounds of formula (I) may also be prepared by first reacting the corresponding carboxylic acid with dicyclohexylcarbodiimide in the presence of 4-dimethylaminopyridine and then treating the product with $R^1OH$. This reaction is conveniently performed at an appropriate temperature (e.g. 0 °C to room temperature) in a solvent such as ether or dichlormethane.

Alternatively, compounds of formula (I) in which the group $-CO_2R^1$ is

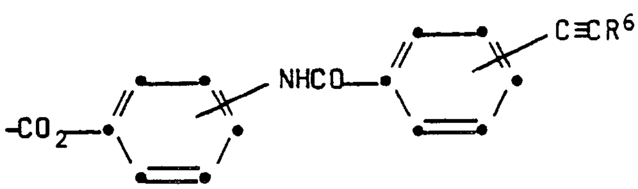

may be prepared from the corresponding acid (i.e. a compound of formula (I) in which the group $-CO_2R^1$ is $-CO_2H$) in a two-step reaction which comprises (i) treating the acid with a reagent

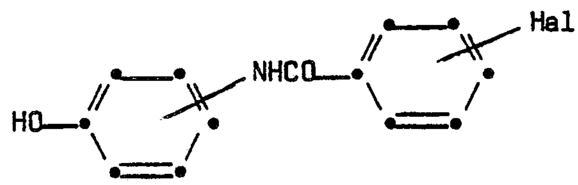

(where Hal is a halogen atom, e.g. iodine) under the esterification conditions described above and (ii) treating the resulting compound with an alkyne $R^6C\equiv CH$ in the presence of CuI and a suitable palladium catalyst such as bis(triphenylphosphine)palladium dichloride conveniently in an ether solvent (e.g. tetrahydrofuran) and in the presence of a suitable organic base such as an amine (e.g. diethylamine).

Intermediate compounds of formula (I) in which $R^1$ represents a hydrogen atom are known compounds described in EP-A-160495 and EP-A-265247.

When a specific enantiomer of formula (I) is required, starting materials having the desired stereochemical configuration should be used in the above processes. Such starting materials may be prepared for example using the methods described in EP-A-160495 from an enantiomeric intermediate as described in EP-A-74856.

Cyclodextrin complexes may be prepared by treating a compound of formula (I) with α-, β- or γ-cyclodextrin in a suitable solvent under conventional conditions and thereafter isolating the product formed.

The skilled chemist will appreciate that solvates (e.g. hydrates) of compounds of the invention may be formed during work-up in the final step conversion reaction.

It will be appreciated that certain intermediates described herein will themselves be novel entities, and it is to be understood that the present invention covers all such novel intermediates.

The following examples are provided by way of illustrating the invention and are not to be construed as constituting a limitation of the invention. In the following examples all temperatures are in °C. Flash chromatography was carried out on silica gel (Merck 9385).

## Intermediate 1

[1S-[1α(Z),2β(2S*), 3α,5α]]-7-[5-Hydroxy-2-[3-phenoxy-2-[(tetrahydro-2H-pyran-2-yl)oxy]propoxy]-3-[-(tetrahydro-2H-pyran-2-yl)oxy]cyclopentyl]-4-heptenoic acid

Intermediate 12f in GB-A-2174702.

## Intermediate 2

5-Hexyn-1-amine

To a stirred solution of lithium aluminium hydride (0,26g) in dry ether (10ml) at room temperature was added 5-hexynitrile (0.5g). The solution was stirred for a further 2h, then water added slowly. Saturated sodium sulphate solution (20ml) was added and the solution extracted with ether (3x50ml), dried ($MgSO_4$) and the solvent removed in vacuo to yield the title compound as a colourless oil (0.37g). I.r. ($CHBr_3$) 3300, 2110cm$^{-1}$.

## Intermediate 3

N-(5-Hexynyl)benzenesulphonamide

To a solution of Intermediate 2 (0.37g) in dry pyridine (10ml) at 0° was added benzenesulphonyl chloride (0.73ml). After 3h at 0° the solution was added to water (50ml), extracted with ether (4x50ml) and the combined organic extracts washed with 10% copper sulphate solution and water and dried ($MgSO_4$).

The solvent was removed in vacuo and the residue purified by flash chromatography eluting with ether:petrol (1:1) to yield the title compound as a white solid (0.40g). M.p. 58-60°.

In a similar manner was prepared Intermediate 4.

Intermediate 4

N-(5-Hexynyl)benzamide

(0.40g). T.l.c. (silica) using ether:petrol (1:1) as solvent gave Rf 0.25.

From Intermediate 2 (0.49g) and benzoyl chloride (0.70ml).

Intermediate 5

5-Hexynamide

To a stirred solution of 5-hexynitrile (0.5g) in dichloromethane (2ml) at 0° was added 30% hydrogen peroxide solution (2ml), tetrabutylammonium hydrogen sulphate (0.29g) and 5M sodium hydroxide solution (1.5ml). After 30min at 0° the solution was warmed to room temperature and stirred for a further 2h, extracted with dichloromethane (3x50ml), dried ($MgSO_4$) and the solvent removed in vacuo. The residue was purified by flash chromatography eluting with ethyl acetate to yield the title compound as a white crystalline solid (0.16g). T.l.c. (silica) using ethyl acetate as solvent gave Rf 0.18.

Intermediate 6

N-(4-Hydroxyphenyl)-4-iodobenzamide

Trimethylacetyl chloride (6.9mℓ) was added to a stirred solution of iodobenzoic acid (10g) and triethylamine (7.9mℓ) in dry tetrahydrofuran (210mℓ). After 30min a solution of 4-aminophenol (10.13g) in dry dimethylformamide (40mℓ) was added. After 18h the suspension was filtered and the filtrate concentrated in vacuo. The residue was dissolved in ethyl acetate (200mℓ) and water (200mℓ) was added. The resulting precipitate was filtered to give the title compound (8.8g) as a white solid. M.p. 284-286°.

Intermediate 7

N-(4-Hydroxyphenyl)-4-[6-[(phenylsulphonyl)amino]-1-hexynyl]benzamide

A suspension of Intermediate 6 (0.63g), Intermediate 3 (0.40g), bis(triphenylphosphine)palladium (II) dichloride (38mg) and copper (1) iodide (5mg) in diethylamine:tetrahydrofuran (15ml of a 4:1 mixture) was stirred under nitrogen for 18h. The solvent was removed in vacuo, the resulting brown tar triturated with dichloromethane and the resulting suspension filtered to give the title compound as a white solid (0.62g). M.p. 185-187°.

In a similar manner were prepared Intermediates 8-11 from Intermediate 6 and an appropriate alkyne.

Intermediate 8

N-(4-Hydroxyphenyl)-4-(phenylethynyl)benzamide

(1.51g). M.p. 276-280°.

From phenylacetylene (0.73mℓ) and Intermediate 6 (2g) except that trituration was effected with isopropanol.

Intermediate 9

4-(1-Hexynyl)-N-(4-hydroxyphenyl)benzamide

(381mg). M.p. 236°.
From hexyne (0.19ml) and Intermediate 6 (500mg).

Intermediate 10

4-[[6-(Benzoylamino)-1-hexynyl]-N-(4-hydroxyphenyl)]benzamide

(240mg). M.p. 210-214°.
From Intermediate 6 (700mg) and Intermediate 4 (400mg).

Intermediate 11

4-(6-Amino-6-oxo-1-hexynyl)-N-(4-hydroxyphenyl)benzamide

(0.57g). M.p. 200-205°.
From Intermediate 6 (0.50g) and Intermediate 5 (0.16g).

Intermediate 12

N-(4-Hydroxyphenyl)-4-[6-[(tetrahydro-2H-pyran-2-yl)oxy]-1-hexynyl]benzamide

A mixture of Intermediate 6 (337mg), 2-(5-hexynyloxy)tetrahydro-2H-pyran (217mg), bis-(triphenylphosphine)palladium (II) chloride (25mg), copper (I) iodide (5mg), diethylamine (5.4mℓ) and tetrahydrofuran (1.4mℓ) was stirred under nitrogen for 18h. The solvent was evaporated in vacuo and the residue partitioned between phosphate buffer (30mℓ, pH 6.5) and ethyl acetate (30mℓ). The mixture was filtered and the organic phase was washed with water and brine and was dried ($Na_2SO_4$). Solvent removal in vacuo gave an oil which was purified by flash chromatography eluting with ethyl acetate:hexane (1:1) to give the title compound as a straw coloured solid (310mg). M.p. 136-8°.

Intermediate 13

N-(4-Hydroxyphenyl)-4-[6-[(phenylsulphonyl)amino]hexyl]benzamide

To a pre-hydrogenated suspension of 5% palladium on carbon (100mg) in ethanol (10ml) was added a solution of Intermediate 7 (0.62g) in ethanol:tetrahydrofuran (20ml of a 1:1 mixture). The suspension was stirred under hydrogen until uptake was complete, filtered through hyflo and the solvent removed in vacuo. The residue was purified by flash chromatography eluting with ethyl acetate:hexane (1:1) to yield the title compound as a white solid (0.17g). T.l.c. (Silica) using ethyl acetate:hexane (1:1) as solvent gave Rf 0.22.

In a similar manner were prepared Intermediates 14 and 15.

Intermediate 14

4-[[(4-Hydroxyphenyl)amino]carbonyl]benzenehexanamide

(0.11g). N.m.r. ($CDCl_3$) 1.30 (2H, m), 1.5-1.7 (4H, m), 2.05 (2H, t), 2.66 (2H, t), 6.7-6.8 (3H, m), 7.25-7.33 (3H, m), 7.53 (2H, m), 7.87 (2H, m), 9.30 (1H, s), 10.0 (1H, s).

From Intermediate 11 (0.18g) except that the purification step was unnecessary.

Intermediate 15

[6-(Benzoylamino)hexyl]-N-(4-hydroxyphenyl)benzamide

(0.17g). I.r. (nujol) 3330, 1638, 1530, 828cm$^{-1}$.

From Intermediate 10 (0.27g) except that the purification step was unnecessary.

Intermediate 16

N-(4-Hydroxyphenyl)-4-(2-phenylethyl)benzamide

A solution of Intermediate 8 (400mg) in tetrahydrofuran:ethanol (2:1, 60mℓ) was added to a pre-hydrogenated suspension of 5% palladium-on-carbon (30mg) in ethanol (20mℓ. After 3h the suspension was filtered and the solvent was removed in vacuo to yield a solid. The solid was submitted to the above conditions again and after 63h the suspension was filtered and solvent removed in vacuo to leave a solid. Recrystallization of this residue from ethyl acetate gave the title compound (300mg) as a buff solid. M.p. 234°.

Intermediate 17

N-(4-Hydroxyphenyl)-4-[6-[(tetrahydro-2H-pyran-2-yl)oxy]hexyl] benzamide

A solution of Intermediate 12 (425mg) in ethanol (75mℓ) was hydrogenated over 5% palladium-on-carbon (200mg). When hydrogen uptake was complete the mixture was filtered through hyflo and the solvent was evaporated in vacuo to give a white solid which was purified by flash chromatography eluting with ethyl acetate:hexane (1:1) to give the title compound as a white solid (360mg). M.p. 125-6°.

Intermediate 18

[1S-[1α(Z),2β(2S*),3α,5α]]-4-[4-[6-[(Phenylsulphonyl)amino]hexyl] benzoylamino]phenyl 7-[5-hydroxy-2-[3-phenoxy-2-[(tetrahydro-2H-pyran-2-yl)oxy]propoxy]-3-[(tetrahydro-2H-pyran-1-yl)oxy]cyclopentyl]-4-heptenoate

A suspension of Intermediate 13 (0.17g), dicyclohexylcarbodiimide (85mg), dimethylaminopyridine (20mg) and Intermediate 1 (0.23g) in dry dichloromethane (5ml) was stirred under nitrogen for 18h. The solvent was removed in vacuo and the residue purified by flash chromatography eluting with 66% ethyl acetate:hexane→ethyl acetate to yield the title compound as a pale yellow gum (0.46g). T.l.c. (silica) using ethyl acetate as solvent gave Rf 0.67.

In a similar manner were prepared Intermediate 19-22 from Intermediate 1 and an appropriate phenol.

Intermediate 19

[1S-[1α(Z),2β(2S*),3α,5α]]-4-[4-(2-Phenylethyl)benzoylamino]phenyl 7-[5-hydroxy-2-[3-phenoxy-2-[(tetrahydro-2H-pyran-2-yl)oxy]propoxy]-3-[(tetrahydro-2H-pyran-2-yl)oxy]cyclopentyl]-4-heptenoate

(310mg). M.p. 58-60°.

From Intermediate 1 (260mg) and Intermediate 16 (168mg) except that ethyl acetate:hexane (1:1) was used during flash chromatography.

Intermediate 20

[1S-[1α(Z),2β(2S*),3α,5α]]-4-[4-[6-[Tetrahydro-2H-pyran-2-yl)oxy] hexyl]benzoylamino]phenyl 7-[5-hydroxy-2-[3-phenoxy-2-[(tetrahydro-2H-pyran-2-yl)oxy]propoxy]-3-[(tetrahydro-2H-pyran-2-yl) oxy]cyclopentyl]-4-heptenoate

(100mg). T.l.c. (silica) using diethyl ether as solvent gave Rf 0.4.

From Intermediate 1 (100mg) and Intermediate 17 (89mg) except that ether (20ml) was added before the solvent removal and ethyl acetate:hexane (11:9) was used during flash chromatography.

Intermediate 21

[1S-[1α(Z),2β(2S*),3α,5α]]-4-[4-[6-(Benzoylamino)hexyl]benzoylamino] phenyl 7-[5-hydroxy-2-[3-phenoxy-2-[(tetrahydro-2H-pyran-2-yl)oxy]propoxy]-3-[(tetrahydro-2H-pyran-2-yl)oxy]cyclopentyl]-4-heptenoate

(53mg). T.l.c. (silica) using ethyl acetate:hexane (1:1) as solvent gave Rf 0.18.

From Intermediate 1 (56 mg) and Intermediate 15 (40 mg) except that ethyl acetate:hexane (1:1) was used during flash chromatography.

Intermediate 22

[1S-[1α(Z),2β(2S*),3α,5α]]-4-(4-Iodobenzoylamino)phenyl 7-[5-hydroxy-2-[3-phenoxy-2-[(tetrahydro-2H-pyran-2-yl)oxy]propoxy]-3-[(tetrahydro-2H-pyran-2-yl)oxy]cyclopentyl]-4-heptenoate

(565 mg). T.l.c. (silica) using ethyl acetate:petrol (1:1) as solvent gave Rf 0.46.

From Intermediate 11 (500 mg) and Intermediate 6 (335 mg) except that the following work-up procedure was followed. After 16h the suspension was poured into water (40mℓ) and extracted with dichloromethane (2x50mℓ). The combined organic extracts were dried ($MgSO_4$), filtered and evaporated to yield a yellow oil. The oil was purified by flash chromatograpy eluting with ethyl acetate:petrol (2:3) to give the title compound.

Intermediate 23

[1S-[1α(Z),2β(2S*),3α,5α]]-4-[4-[(6-Amino-6-oxo-hexyl]benzoylamino] phenyl 7-[5-hydroxy-2-[3-phenoxy-2-[(tetrahydro-2H-pyran-2-yl)oxy] propoxy-3-[(tetrahydro-2H-pyran-2-yl)oxy]cyclopentyl]-4-heptenoate

Pivaloyl chloride (45mg) was added to a solution of Intermediate 1 (0.26g) and triethylamine (0.1mℓ) in dry dimethylformamide (2mℓ) at 0°. The mixture was stirred at 0° for 10min and a solution of Intermediate 14 (0.13g) in dry dimethylformamide (1mℓ) added. The solution was allowed to warm to room temperature and stirred for a further 20h, added to water (50mℓ) and extracted with ethyl acetate 3x50mℓ), dried

($MgSO_4$) and the solvent removed in vacuo to yield an oil. The oil was purified by flash chromatography eluting with ethyl acetate to yield the title compound as a colourless oil (0.15g). I.r. ($CHBr_3$) 3520, 3400, 1745, 1675cm$^{-1}$.

Intermediate 24

[1S-[1α(Z),2β(2S*),3α,5α]]-4-[4-(Phenylethynyl)benzoylamino]phenyl 7-[5-hydroxy-2-[3-phenoxy-2-[(tetrahydro-2H-pyran-2-yl)oxy]propoxy]-3-[(tetrahydro-2H-pyran-2-yl)oxy]cyclopentyl]-4-heptenoate

Phenylacetylene (0.075mℓ) was added to a stirred solution of copper (I) iodide (30mg), bis-(triphenylphosphine)palladium dichloride (77mg) and Intermediate 22 (396mg) in diethylamine:tetrahydrofuran (4:1, 10mℓ) under a nitrogen atmosphere. After 3 days the mixture was concentrated in vacuo to leave a dark brown oil. The oil was purified by flash chromatography eluting with ethyl acetate:hexane (1:1) to give the title compound (285mg) as a buff coloured solid. M.p. 84-86°.

In a similar manner was prepared Intermediate 25.

Intermediate 25

[1S-[1α(Z),2β(2S*),3α,5α]]-4-[4-(1-Hexynyl)benzoylamino]phenyl 7-[5-hydroxy-2-[3-phenoxy-2-[(tetrahydro-2H-pyran-2-yl)oxy]propoxy]-3-[(tetrahydro-2H-pyran-2-yl)oxy]cyclopentyl]-4-heptenoate

(269 mg). T.l.c. (silica) using ethyl acetate:hexane (1:1) as solvent gave Rf 0.26.

From Intermediate 22 (436 mg) and 1-hexyne (0.17mℓ).

Intermediate 26

[1R-[1α(Z),2β(2R*),3α,5α]]-4-[4-[6-Amino-6-oxo-hexyl]benzoylamino]phenyl 7-[5-oxo-2-[3-phenoxy-2-[(tetrahydro-2H-pyran-2-yl)oxy]propoxy]-3-[(tetrahydro-2H-pyran-2-yl)oxy]cyclopentyl]-4-heptenoate

To a solution of Intermediate 23 (0.15g) in dry dichloromethane (5mℓ) was added sodium acetate (42mg), pyridinium chlorochromate (0.11g) and powdered 3Å molecular sieves (50mg) at 0° under nitrogen. the solution was stirred for 4h, ethyl acetate (100mℓ) added and the suspension filtered through hyflo. The solvent was removed in vacuo and the residue purified by flash chromatography (Merck 9385, acid washed to pH4) eluting with ethyl acetate to yield the title compound as a pale yellow gum (60mg). T.l.c. (silica) using ethyl acetate as solvent gave Rf 0.35.

Intermediate 27

[1R-[1α(Z),2β(2R*),3α]]-4-[4-[6-[(Phenylsulphonyl)amino]hexyl] benzoylamino]phenyl 7-[5-oxo-2H-[3-phenoxy-2-[(tetrahydro-2H-pyran-2-yl)oxy]propoxy]-3-[(tetrahydro-2H-pyran-2-yl)oxy]cyclopentyl]-4-heptenoate

A solution of dicyclohexylcarbodiimide (1.0g) in dry dichloromethane (1mℓ) was added to a stirred solution of Intermediate 18 (0.46g) in dry dichloromethane (10mℓ) and dimethylsulphoxide (1.0mℓ). Pyridinium trifluoroacetate (0.47g) was added and the solution stirred for 18h. The solution was filtered through hyflo which was washed with dichloromethane. The filtate was washed with 10% copper sulphate solution, the aqueous washings were back-extracted with dichloromethane (2x30mℓ) and the combined organic extracts dried ($MgSO_4$). The solvent was removed in vacuo and the residue purified by flash chromatography (Merck 9385, acid washed to pH4) eluting with ethyl acetate:hexane (1:1) to yield the title compound as a pale yellow oil (0.32g). T.l.c. (silica) using ethyl acetate:hexane (1:1) as solvent gave Rf

0.18.

In a similar manner was prepared Intermediate 28.

Intermediate 28

[1R-[1α(Z),2β(2R*),3α]]-4-[4-[6-(Benzoylamino)hexyl]benzoylamino] phenyl 7-[5-oxo-2-[3-phenoxy-2-[-(tetrahydro-2H-pyran-2-yl)oxy] propoxy]-3-[(tetrahydro-2H-pyran-2-yl)oxy]cyclopentyl]-4-heptenoate

(0.32g). T.l.c. (silica) using ethyl acetate:hexane (1:1) as solvent gave Rf 0.25.

From Intermediate 21 (0.35g).

Intermediate 29

[1R-[1α(Z),2β(2R*),3α]]-4-[4-[6-[(Tetrahydro-2H-pyran-2-yl)oxy]hexyl] benzoylamino]phenyl 7-[5-oxo-2-[3-phenoxy-2-[(tetrahydro-2H-pyran-2-yl)oxy]propoxy]-3-[(tetrahydro-2H-pyran-2-yl)oxy]cyclopentyl]-4-heptenoate

A solution of dicyclohexylcarbodiimide (714mg) in dry dichloromethane (2mℓ) was added to a mixture of Intermediate 20 (326mg) and dimethylsulphoxide (823mg) in dry dichloromethane (10mℓ) under nitrogen. Pyridinium trifluoroacetate (334mg) was added and the mixture was stirred at room temperature for 4h. Dichloromethane (20mℓ) was added and the mixture was filtered through hyflo. The filtrate was evaporated in vacuo and the residue was triturated with ether (20mℓ) and filtered. The filtrate was evaporated in vacuo and the residue was purified by flash chromatograpy (Merck 9385, acid washed to pH4) eluting with diethyl ether:hexane (3:1) to give the title compound as a colourless oil (290mg). T.l.c. (silica using diethyl ether as solvent gave Rf 0.5.

Intermediate 30

[1R-[1α(Z),2β(2R*),3α,]]-4-[4-(1-Hexynyl)benzoylamino]phenyl 7-[5-oxo-2-[3-phenoxy-2-[(tetrahydro-2H-pyran-2-yl)oxy]propoxy]-3-[(tetrahydro-2H-pyran-2-yl)oxy]cyclopentyl]-4-heptenoate

Pyridinium trifluoroacetate (220mg) was added to a stirred solution of Intermediate 25 (256mg) and dicyclohexylcarbodiimide (386mg) in dimethylsulphoxide (0.55mℓ) and dry dichloromethane (15mℓ). Stirring was continued for 20h, then further quantities of dicyclohexylcarbodiimide (380mg) and pyridinium trifluoroacetate (218mg) were added. After a further 6h water (20mℓ) was added and the mixture filtered through hyflo and concentrated in vacuo to leave a solid. The solid was dissolved in ether and washed with water, 10% copper sulphate solution and brine. The aqueous washings were back-extracted with ether and the combined organic extracts dried ($MgSO_4$), filtered and evaporated to yield a yellow solid. The solid was purified by flash chromatography (Merck 9385, acid washed to pH4) eluting with ethyl acetate:hexane (1:2) to give the title compound (212mg) as a yellow oil. T.l.c. (silica) using ethyl acetate:hexane (1:1) as solvent gave Rf 0.5.

In a similar manner were prepared Intermediate 31 and 32.

Intermediate 31

[1R-[1α(Z),2β(2R*),3α]]-4-[4-(2-Phenylethyl)benzoylamino]phenyl 7-[5-oxo-2-[3-phenoxy-2-[(tetrahydro-2H-pyran-2-yl)oxy]propoxy]-3-[(tetrahydro-2H-pyran-2-yl)oxy]cyclopentyl]-4-heptenoate

(207mg). T.l.c. (silica) using ethyl acetate:hexane (1:1) as solvent gave Rf 0.37.

From Intermediate 19 (289mg) except that ethyl acetate:hexane (2:3) was used as eluent during flash

chromatography.

Intermediate 32

[1R-[1α(Z),2β(2R*),3α]]-4-[4-(Phenylethynyl)benzoylamino]phenyl 7-[5-oxo-2-[3-phenoxy-2-[(tetrahydro-2H-pyran-2-yl)oxy]propoxy]-3-[(tetrahydro-2H-pyran-2-yl)oxo]cyclopentyl]-4-heptenoate

(182mg). T.l.c. (silica) using ethyl acetate:hexane (1:1) as solvent gave Rf 0.55.

From Intermediate 24 (253mg) except that ethyl acetate:hexane (2:3) was used as eluent during flash chromatography.

Example 1

[1R-[1α(Z),2β(2R*),3α]]-4-[4-[6-[(Phenylsulphonyl)amino]hexyl] benzoylamino]phenyl 7-[3-hydroxy-2-(2-hydroxy-3-phenoxypropoxy)-5-oxocyclopentyl]-4-heptenoate

A solution of Intermediate 27 (0.32g) in acetic acid:water:tetrahydrofuran (7ml of a 6:3:1 solution) was stirred at 40° for 5h. The solvent was removed in vacuo and the residue purified by flash chromatography (Merck 9385, acid washed to pH4) eluting with ethyl acetate:hexane (3:1) to yield the title compound as a white foam (0.12g). T.l.c. (silica) using ethyl acetate as solvent gave Rf 0.4.

| | | | |
|---|---|---|---|
| Analysis Found : | C,68.10; | H,7.17; | N,3.4. |
| $C_{46}H_{54}N_2O_{10}S$ requires : | C,66.81; | H,6.58; | N,3.39%. |

Examples 2-7 were prepared in a similar manner.

Example 2

[1R-[1α(Z),2β(2R*),3α]]-4-[4-[6-Amino-6-oxo-hexyl]benzoylamino]phenyl 7-[3-hydroxy-2-(2-hydroxy-3-phenoxypropoxy)-5-oxocyclopentyl]-4-heptenoate

(26mg). T.l.c. (silica) using 5% methanol: ethyl acetate as solvent gave Rf 0.24. I.r. ($CHBr_3$) 3410, 1740, 1680cm$^{-1}$.

From Intermediate 26 (60mg) except that the eluent used in the flash chromatography was 5% methanol:ethyl acetate.

Example 3

[1R-[1α(Z),2β(2R*),3α]]-4-[4-[6-(Benzoylamino)hexyl]benzoylamino] phenyl 7-[3-hydroxy-2-(2-hydroxy-3-phenoxypropoxy)-5-oxocyclopentyl]-4-heptenoate

(80mg). M.p. 100-104°.

| | | | |
|---|---|---|---|
| Analysis Found: | C,70.72; | H,6.89; | N,3.45. |
| $C_{47}H_{54}N_2O_9$ requires | C,71.37; | H,6.88; | N,3.54%. |

From Intermediate 28 (320mg) except that gradient elution (75% ethyl acetate:hexane → 100% ethyl acetate) was used in the flash chromatography.

Example 4

[1R-[1α(Z),2β(2R*),3α]]-4-[4-(1-Hexynyl)benzoylamino]phenyl 7-[3-hydroxy-2-(2-hydroxy-3-phenoxypropoxy)-5-oxocyclopentyl]-4-heptenoate

(87mg). M.p. 98-100°.

| | | | |
|---|---|---|---|
| Analysis Found: | C,71.58; | H,6.96; | N,1.97. |
| $C_{40}H_{45}NO_8$ requires | C,71.94; | H,6.79; | N,2.1%. |

From Intermediate 30 (186mg) except that the flash chromatography was carried out twice using ethyl acetate:hexane (2:1) as eluent.

Example 5

[1R-[1α(Z),2β(R*),3α]]-4-[4-(2-Phenylethyl)benzoylamino]phenyl 7-[3-hydroxy-2-(2-hydroxy-3-phenoxypropoxy)-5-oxocyclopentyl]-4-heptenoate

(93mg). T.l.c. (silica) using ethyl acetate:hexane (3:1) as solvent gave Rf 0.34.

| | | | |
|---|---|---|---|
| Analysis Found : | C,72.04; | H,6.59; | N,1.90. |
| $C_{42}H_{45}NO_8$ requires | C,72.92; | H,6.56; | N,2.02%. |

From Intermediate 31 (182mg).

Example 6

[1R-[1α(Z),2β(R*),3α]]-4-[4-(6-Hydroxyhexyl)benzoylamino]phenyl 7-[3-hydroxy-2-(2-hydroxy-3-phenoxypropoxy)-5-oxocyclopentyl]-4-heptenoate

(94mg). M.p. 66-68°.

| | | | |
|---|---|---|---|
| Analysis Found: | C,69.7; | H,7.3; | N,1.9. |
| $C_{40}H_{49}NO_9$ requires | C,69.85; | H,7.2; | N,2.0%. |

From Intermediate 29 (260mg) except that the eluent used in the flash chromatography was ethyl acetate and the resulting foam was triturated with ether to give the title compound.

Example 7

[1R-[1α(Z),2β(R*),3α]]-4-[4-(Phenylethynyl)benzoylamino]phenyl 7-[3-hydroxy-2-(2-hydroxy-3-phenoxypropoxy)-5-oxocyclopentyl]-4-heptenoate

(79mg). M.p. 152-155°.

| | | | |
|---|---|---|---|
| Analysis Found: | C,72.96; | H,6.07; | N,1.91 |
| $C_{42}H_{41}NO_8$ requires | C,73.34; | H,6.00; | N,2.03%. |

From Intermediate 32 (164mg) except that the eluent used in the flash chromatography was ethyl acetate:hexane (2:1).

The following are examples of pharmaceutical formulations using compounds of the invention. In the examples, the term "active ingredient" is used to denote a compound of the invention, such as a compound described in the preceding examples.

| 1. Tablets | |
|---|---|
| These may be prepared by direct compression | |
| | mg/tablet |
| Active Ingredient | 0.015 to 0.2 |
| Magnesium stearate, BP | 1.5 |
| Microcrystalline cellulose, USP to compression weight | 150.0 |

The active ingredient is blended with about 10% of the microcrystalline cellulose then blended with the remaining microcrystalline cellulose and magnesium stearate. The blend is then compressed using 6mm diameter punches into tablets on a suitable machine.

The tablets may be film coated with suitable film forming materials e.g. methyl cellulose or hydroxypropyl methylcellulose using standard techniques.

| 2. Capsules | |
|---|---|
| | mg/tablet |
| Active ingredient | 0.015 to 0.2 |
| Magnesium stearate, BP | 1.0 |
| *Starch 1500 to fill weight | 100.0 |

*A form of directly compressible starch.

The active ingredient is preblended with some of the Starch 1500 then this preblend is mixed with the remaining Starch 1500 and magnesium stearate. The mix is then filled into size No 2 hard gelatin capsule shells using suitable machinery.

## Claims

1. Compounds of the general formula (I)

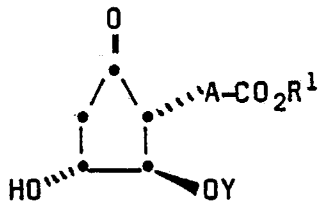

(I)

and the physiologically acceptable solvates and cyclodextrin complexes thereof, wherein

$R^1$ represents phenyl substituted by a group $-NHCOR^5$ [where $R^5$ is phenyl substituted by a group selected from $-C\equiv CR^6$ (where $R^6$ is hydrogen, $C_{1-6}$ alkyl or phenyl), phenyl$C_{1-6}$alkyl or $C_{1-10}$alkyl optionally substituted by hydroxy, $-NHSO_2R^7$, $-NHCOR^7$ or $-CONR^8R^9$ (where $R^7$ is $C_{1-4}$alkyl or phenyl and $R^8$ and $R^9$ may be the same or different and are each a hydrogen atom or a $C_{1-4}$ alkyl group)];

A represents a group $-(CH_2)_nX(CH_2)_m-$ or a group $-(CH_2)_pS(O)_q(CH_2)_r-$ (where n is 1 or 2, m is 2-5 and X is cis or trans $-CH=CH-$ or $-CH_2CH_2-$ or m is 1-4 and X is $-CH=C=CH-$; p is 1-4, q is zero, 1 or 2 and r is 1-4); and

Y is

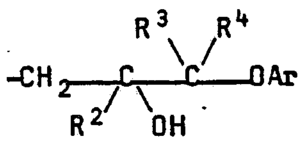

where $R^2$, $R^3$ and $R^4$ are each a hydrogen atom or a methyl group and at least one of $R^2$, $R^3$ and $R^4$ is a hydrogen atom and Ar represents a phenyl group optionally substituted by one or two $C_{1-4}$alkyl, $C_{1-4}$alkoxy, $C_{1-4}$alkylthio, $C_{1-4}$alkylsulphinyl, $C_{1-4}$alkylsulphonyl, halogen or trifluoromethyl groups).

2. Compounds as claimed in Claim 1 in which A represents a group $-CH_2)_nX(CH_2)_m-$ where X is $-CH=CH-$ or $-CH_2CH_2-$ and m is 3 when n is 1 and m is 2 or 4 when n is 2; or X is $-CH=C=CH-$ and m is 2 when n is 1 and m is 1 or 3 when n is 2.

3. Compounds as claimed in Claim 1 in which A represents a group $-(CH_2)_pS(O)_q(CH_2)_r-$, where q is zero and p is 3 or 4 when r is 1 and p is 2 or 3 when r is 2 and p is 2 when r is 3 and p is 1 when r is 4.

4. Compounds as claimed in any preceding claim in which $R^5$ represents phenyl substituted by $-C\equiv CR^6$ (where R6 is $C_{1-4}$alkyl or phenyl), phenyl$C_{1-3}$alkyl or $C_{1-10}$alkyl substituted by hydroxy, $-NHSO_2R^7$ - (where $R^7$ is phenyl), $-NHCOR^7$ (where $R^7$ is phenyl) or $-CONH_2$.

5. Compounds as claimed in any preceding claim in which $R^3$ and $R^4$ are hydrogen atoms and Ar is phenyl or phenyl substituted by fluoro or chloro.

6. Compounds as claimed in Claim 1 in which :

$R^1$ represents phenyl substituted in the para position by a group $-NHCOR^5$ [where R5 represents phenyl substituted in the para position by $-C\equiv CR^6$ (where R6 is butyl or phenyl), phenyl$C_{1-3}$alkyl or $C_{4-8}$alkyl substituted by hydroxy, $-NHSO_2R^7$ (where R7 is phenyl), -NHCOR7 (where $R^7$ is phenyl) or $-CONH_2$];

A represents $-CH_2CH=CH(CH_2)_3-$ or $-CH_2CH_2CH=CHCH_2CH_2-$; and

Y represents

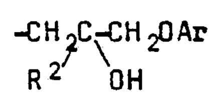

where $R^2$ is a hydrogen atom or a methyl group and Ar is phenyl or phenyl substituted by fluoro or chloro).

7. Compounds as claimed in Claim 6 in which A represents $-CH_2CH_2CH=CHCH_2CH_2-$ wherein the double bond is in the cis configuration.

8. Compounds as claimed in any preceding claim in which the carbon atom carrying the group $-A-CO_2R^1$ is in the R-configuration.

9. A pharmaceutical composition comprising a compound as claimed in any preceding claim together with one or more pharmaceutical carriers.

10. A compound as claimed in any one of Claims 1 to 8 for use in therapy.

11. Use of a compound as claimed in any one of Claims 1 to 8 in the manufacture of a medicament for

the prevention or treatment of ulcers and other conditions arising from hypersecretion of gastric acid.

12. Use of a compound as claimed in any one of Claims 1 to 8 in the manufacture of a medicament for the prevention or treatment of atherosclerosis and other diseases associated with lipid imbalance or abnormalities in lipid mechanism.

13. A process for the preparation of a compound as claimed in Claim 1 which comprises :

(a) deprotecting a corresponding compound in which the ring hydroxy group and the hydroxy group in Y are protected; or

(b) esterifying the corresponding carboxylic acid (i.e. a compound of formula (I) in which $R^1$ is a hydrogen atom).